# EUROPEAN PATENT APPLICATION

(11) **EP 3 572 467 A1**
(43) Date of publication of application: **27.11.2019**
(21) Application number: 18741641.7
(22) Date of filing: 19.01.2018
(51) Int. Cl.: C08L 101/02, A61K 8/19, A61K 8/24, A61K 8/27, A61K 8/81, A61Q 1/00, A61Q 19/00, C08K 3/02, C08K 3/22

(54) **POWDER-CONTAINING COMPOSITION, PRODUCTION METHOD THEREFOR, AND COSMETIC PREPARATION**

(30) Priority: 23.01.2017 JP 2017009157
(71) Applicant: Shiseido Company, Ltd., Tokyo 104-0061 (JP)
(72) Inventor: NASU Akio, Yokohama-shi Kanagawa 224-8558 (JP); SATONE Hiroshi, Himeji-shi Hyogo 671-2280 (JP)
(74) Representative: Ter Meer Steinmeister & Partner
(86) International application number: PCT/JP2018/001618
(87) International publication number: WO 2018/135626

(57) **Abstract**

A composition includes a solvent, a powder that is present in the solvent and bears a first charge on its surface, an ionic polymer having a second charge that is opposite to the first charge, and a polyvalent ion having the first charge. The powder and the ionic polymer are electrostatically and/or ionically bonded with each other. The ionic polymer and the polyvalent ion are electrostatically and/or ionically bonded with each other.

## Description

### Technical Field

The present disclosure relates to a liquid composition comprising a powder in a solvent and a production method thereof. Furthermore, the present disclosure relates to a cosmetic comprising the aforementioned composition.

### Background Art

Slurry products in which solid particles are dispersed in a solvent are used in a wide field such as foods, paints, cosmetics, inks, ceramics and the like. Technique for enhancing dispersibility of particles has been developed for such products (for example, Patent Literature 1 and Non-patent Literature 1).

A dispersion containing organic-inorganic composite particles described in Patent Literature 1 is prepared by dispersing the organic-inorganic composite particles in a range of 0.001 to 50% by weight in a solvent selected from oils and fats, waxes, hydrocarbons, fatty acids, alcohols, alkyl glyceryl ethers, esters, polyhydric alcohols, saccharides, silicone oil, crosslinked silicone gel and fluorine oil, or a mixed solvent thereof. The aforementioned organic-inorganic composite particle is used for cosmetics of which a polymeric gel molecule derived from a natural substance and having an anionic functional group and one or more hydroxyl groups in a molecule is electrostatically bonded to a surface of an inorganic oxide particle having a cationic charge on the particle surface.

In Non-patent Literature 1, ammonium polycarboxylate is used as a polymeric dispersant to prepare a slurry having alumina particles dispersed in ion-exchanged water. In Non-patent Literature 1, the polymer chain adsorbed to the particle surface is contracted in a coiled state.

### Citation List

### Patent Literature

Patent Literature 1: Japanese Patent Application Laid Open Publication No. 2011-51972 A.

### Non-Patent Literature

Non-Patent Literature 1: SATONE Hiroshi et al., Effect of Conformation of Adorbed Polymeric Dispersant on Dispersion Stability of Slurry, Journal of the Society of Powder Technology, Japan, 51, 2014, p. 269-274

### Summary of Invention

### Technical Problem

In liquid cosmetics comprising powder for example, it is desired that the powder is dispersed in a solvent or that the user can easily redisperse the powder by a simple action such as shaking the container and the like so that the user may use the aforementioned cosmetic with uniform composition at all times. In cases where the particles aggregate easily, however, it is generally difficult to redisperse the powder since the powder solidifies at the bottom of the container or sticks to the bottom of the container.

The aforementioned slurry products are usually prepared so that the ratio between the solvent and the powder is optimized. In most cases, however, there is a density difference between the solvent and the powder. Therefore, there is a problem that the powder sediments and then forms a solidified deposition layer when it is allowed to stand, and as a result, a function of the product fails. A technique in terms of kinetics has often been used to address this problem, in which the solid particles are crushed to sizes of a few tens of nm or less to make the sedimentation velocity extremely small. However, a complicated dispersion process and addition of additives are necessary to maintain the powder having a particle size of a few tens of nm or less in a stable dispersed state, and thus leads to increase in cost. Furthermore, since this technique just allows the sedimentation velocity to be extremely, complete prevention of solidification of the powder which is allowed to stand over a long time has not been accomplished. Accordingly, there is a demand for a solution for making redispersion easier cost-effectively, simply and fundamentally.

In the slurry described in Non-patent Literature 1, polymers are not stretched sufficiently so that polymers crosslink between non-adsorbed surfaces of the particles and form aggregates. Consequently, the problem of powder solidification as described above occurs. It is considered that aggregation like in the slurry described in Non-patent Literature 1 occurs in the dispersion containing organic-inorganic composite particles described in Patent Literature 1. Redispersion of the powder becomes difficult when such aggregation occurs.

Hence, there is a demand for a composition in which the powder is hard to solidify and easy to be redispersed.

### Solution to Problem

According to a first aspect of the present disclosure, a composition is provided, the composition comprising a solvent, a powder that is present in the solvent and bears a first charge on a surface, an ionic polymer having a second charge that is opposite to the first charge, and a polyvalent ion having the first charge. The powder and the ionic polymer are electrostatically and/or ionically bonded with each other. The ionic polymer and the polyvalent ion are electrostatically and/or ionically bonded with each other.

According to a second aspect of the present disclosure, a composition is provide, the composition comprising powder bearing a first charge on a surface, a polyacrylate ion and/or a hexametaphosphate ion, and a magnesium ion. A content of the powder is 10% by mass to 50% by mass with respect to the composition. A content of a polyelectrolyte that is a source of the polyacrylate ion and/or the hexametaphosphate ion is 0.005 part by mass to 0.5 part by mass with respect to 1 part by mass of the powder. A content of a salt that is a source of the magnesium ion is 0.05 part by mass to 5 parts by mass with respect to 1 part by mass of the polyelectrolyte.

According to a third aspect of the present disclosure, a cosmetic is provided, the cosmetic comprising the composition according to the first aspect and/or second aspect.

According to a fourth aspect of the present disclosure, a method of producing a composition is provided, the method comprising a first addition step of adding a powder to a solvent comprising water, the powder bearing a first charge on a surface in the solvent; a second addition step of adding a polyelectrolyte to a solvent, wherein the polyelectrolyte ionizes an ionic polymer having a second charge opposite to the first charge in the solvent; and a third addition step of adding a salt to the solvent after the first and the second addition steps, wherein the salt generates a polyvalent ion having the first charge in the solvent.

According to a fifth aspect of the present disclosure, a method of producing a composition is provided, the method comprising a first addition step of adding a powder to a solvent comprising water, the powder bearing a first charge on a surface in the solvent; a second addition step of adding a polyelectrolyte to the solvent, wherein the polyelectrolyte ionizes an ionic polymer having a second charge opposite to the first charge in the solvent; a charging step of charging the surface of the powder with the first charge in the solvent; and a third addition step of adding a salt to the solvent after the charging step, wherein the salt generates a polyvalent ion having the first charge in the solvent.

### Advantageous Effects of Invention

The sedimentation velocity of the powder in the composition of the present disclosure is slow. The powder can be easily redispersed by shaking the container lightly even when the powder is sedimented after being left to stand. That is, the composition of the present disclosure is highly stable upon storing and easily flowable upon use.

### Brief Description of Drawings

FIG. 1 is a schematic view for describing a structure in the composition of the present disclosure.
FIG. 2 shows photographs of samples in Test Examples 1-1 to 1-6, Test Examples 2-1 to 2-6 and Test Examples 3-1 to 3-6.
FIG. 3 shows photographs of samples in Test Examples 1-3, 2-3 and 3-3.
FIG. 4 is a graph showing the relationship between shearing velocity and shearing stress in Test Examples 1-1 to 1-6.
FIG. 5 is a graph showing the relationship between shearing velocity and shearing stress in Test Examples 2-1 to 2-6.
FIG. 6 is a graph showing the relationship between shearing velocity and shearing stress in Test Examples 3-1 to 3-6.

### Description of Embodiments

Preferred modes of the above aspects will be described below.

According to a preferred mode of the first aspect, the powder configures a weak flocculation via the ionic polymer and the polyvalent ion.

According to a preferred mode of the first aspect, the average particle size of a complex having the powder and the ionic polymer attached to the powder is 200 nm to 800 nm.

According to a preferred mode of the first aspect, the first charge is a positive charge, and the second charge is a negative charge.

According to a preferred mode of the first aspect, the powder is zinc oxide.

According to a preferred mode of the first aspect, the content of the powder is 10% by mass to 50% by mass with respect to the mass of the composition.

According to a preferred mode of the first aspect, the average particle size of the powder is 10 nm to 200 nm.

According to a preferred mode of the first aspect, the ionic polymer has an anionic functional group.

According to a preferred mode of the first aspect, the ionic polymer is a polyacrylate ion and/or a hexametaphosphate ion.

According to a preferred mode of the first aspect, the content of a polyelectrolyte that is a source of the ionic polymer is 0.005 part by mass to 0.5 part by mass with respect to 1 part by mass of the powder.

According to a preferred mode of the first aspect, the polyvalent ion is a magnesium ion and/or a calcium ion.

According to a preferred mode of the first aspect, the content of a salt that is a source of the polyvalent ion is 0.05 part by mass to 5 parts by mass with respect to 1 part by mass of the polyelectrolyte that is a source of the ionic polymer.

According to a preferred mode of the first aspect, the solvent comprises water.

According to a preferred mode of the second aspect, the powder is zinc oxide.

According to a preferred mode of the fourth and fifth aspects, pH of the solvent is adjusted in the charging step.

According to a preferred mode of the fourth and fifth aspects, a charging agent that charges the surface of the powder with the first charge is added to the solvent in the charging step.

According to a preferred mode of the fourth and fifth aspects, the charging agent is a cationic polymer.

According to a preferred mode of the fourth and fifth aspects, the method further comprises a loosening step of loosening contraction of a polymer chain of the ionic polymer.

According to a preferred mode of the fourth and fifth aspects, pressure is applied to the solvent in the loosening step.

According to a preferred mode of the fourth and fifth aspects, the powder is zinc oxide.

According to a preferred mode of the fourth and fifth aspects, an average particle size of the powder is 10 nm to 200 nm.

According to a preferred mode of the fourth and fifth aspects, the polyelectrolyte is polyacrylate and/or hexametaphosphate.

According to a preferred mode of the fourth and fifth aspects, the salt is a magnesium salt and/or a calcium salt.

A composition of the present disclosure according to a first embodiment will be described.

In the following description, POE is an abbreviation of polyoxyethylene, POP is an abbreviation of polyoxypropylene, and the number in parentheses following POE or POP indicates the average number of moles of POE groups or POP groups added in the respective compound.

The composition of the present disclosure comprises a solvent, a powder present in the solvent, an ionic polymer (a polymeric ion generated from the ioinc polymer) and a polyvalent ion.

### [Solvent]

The solvent is preferably a liquid capable of dissolving the ionic polymers and metal salts that dissociate the polyvalent ion. The solvent is preferably a polar solvent so that ionic substances can be dissolved, and more preferably an aqueous solvent. Examples of the aqueous solvent may include water, alcohol or a mixture thereof.

Examples of the lower alcohol may include ethanol, propanol, isopropanol, isobutyl alcohol, t-butyl alcohol and the like.

Examples of the polyhydric alcohol may include dihydric alcohol (such as ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc); trihydric alcohol (such as glycerin, trimethylolpropane, etc); tetrahydric alcohol (such as such as pentaerythritol such as 1,2,6-hexanetriol, etc); pentahydric alcohol (such as xylitol, etc); hexahydric alcohol (such as sorbitol, mannitol, etc); polyhydric alcohol polymer (such as diethylene glycol, dipropylene glycol, triethylene glycol, polypropylene glycol, tetraethylene glycol, diglycerin, polyethylene glycol, triglycerin, tetraglycerin, polyglycerin, etc); dihydric alcohol alkyl ethers (such as ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether, etc); dihydric alcohol alkyl ethers (such as diethylene glycol monomethyl ether, diethylene glycol monoethyl ether, diethylene glycol monombutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc); dihydric alcohol ether ethers (such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disaccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc); glycerin monoalkyl ether (such as chimyl alcohol, selachyl alcohol, batyl alcohol, etc); sugar alcohol (such as sorbitol, maltitol, maltotriose, mannitol, sucrose, erythritol, glucose, fructose, starch sugar, maltose, xylitose, starch sugar hydrogenated alcohol, etc); glycolide, tetrahydrofurfuryl alcohol; POE-tetrahydrofurfuryl alcohol; POP/POE-butyl ether; tripolyoxypropylene glycerin ether; POP-glycerin ether; POP-glycerin ether phosphoric acid; POP/POE-pentaerythritol ether; polyglycerin and the like.

The content of the solvent can be the remainder of other components. For example, the solvent may be 40% by mass or more, 50% by mass or more, 60% by mass or more, 70% by mass or more, 80% by mass or more or 90% by mass or more with respect to the mass of the composition. The solvent may be 95% by mass or less, 90% by mass or less, 80% by mass or less, 70% by mass or less or 60% by mass or less with respect to the mass of the composition.

### [Powder]

The composition can comprise a powder. The composition may comprise a plurality of kinds of particles. The powder is preferably insoluble to the solvent. The powder bears a first charge on its surface in the solvent. The first charge may be a charge charged by a charging agent. The first charge may be positive or negative. Analysis and evaluation for checking whether the powder bears the first charge can be performed by measurement of an isoelectric point, measurement of pH and the like. Examples of the powder bearing positively charges on its surface may include zinc oxide, alumina, titanium oxide and the like. Examples of the powder bearing negative charges on its surface may include silica and the like.

The average particle size of the powder is preferably 5 nm or greater, and more preferably 10 nm or greater. If the average particle size is less than 5nm, handling will be difficult. The average particle size of the powder may be preferably 200 nm or smaller or 100 nm or smaller. The average particle size of the powder can be measured in accordance with a dynamic light scattering method.

The content of the powder may be 5% by mass or more, 10% by mass or more or 20% by mass or more with respect to the mass of the composition. Furthermore, the content of the powder may be 50% by mass or less or 40% by mass or less with respect to the mass of the composition.

Examples of the powder bodies may include inorganic powder (such as talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, glass, barium sulfate, calcined calcium sulfate (calcined gypsum), calcium phosphate, fluorine apatite, hydroxyapatite, ceramic powder, metallic soap (such as zinc myristate, calcium palimitate, and aluminum stearate), and boron nitride, etc); organic powder (such as polyamide resin powder (nylon powder), polyethylene powder, polymethylmethacrylate powder, polystyrene powder, styrene-acrylic acid copolymer powder, benzoguanamine resin powder, poly(tetrafluroethylene) powder, and cellulose powder, silicone resin powder, silk powder, wool powder, urethane powder, etc); inorganic white family pigment (such as titanium dioxide, zinc oxide, etc); inorganic red family pigment (such as iron oxide (colcothar), iron titanate, etc); inorganic brown family pigment (such as γ-iron oxide, etc); inorganic yellow family pigment (such as yellow iron oxide, loess, etc); inorganic black family pigment (such as black iron oxide, carbon black, lower titanium oxide, etc); inorganic purple family pigment (such as manganese violet, cobalt violet, etc); inorganic green family pigment (such as chrome oxide, chrome hydroxide, cobalt titanate, etc); inorganic blue family pigment (such as ultramarine, iron blue, etc); pearl pigment (such as titanium oxide coated mica, titanium oxide coated bismuth oxychloride, titanium oxide coated talc, colored titanium oxide coated mica, bismuth oxychloride, argentine, etc); metal powder pigment (such as aluminum powder, copper powder, etc); organic pigment such as zirconium, barium, or aluminum lake (such as organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401, Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1, etc); natural pigment (such as chlorophyll, β-carotene, etc) and the like.

### [Ionic polymer]

The composition of the present disclosure comprises at least one kind of an ionic polymer. The ionic polymer may be a polymer that becomes an ion in the solvent. Positive/negative of the charge of the ionic polymer is preferably determined in accordance with the charge of the powder. One ionic polymer can have one or more ionic functional group(s). Valence of the ionic polymer may be monovalent or polyvalent. The ionic polymer can be added in a form of a polyelectrolyte. The polyelectrolyte can be in a form of an acid or a base.

The ionic polymer bears a second charge opposite to the first charge by ionization in the solvent. In a constituent or a substituent of a polymer chain, the ionic polymer has one or more of an ionic functional group that ionizes in the solvent and has the second charge. Valence of one functional group is preferably lower than that of the polyvalent ion. In order to electrostatically bond and/or ionically bond a plurality of the ionic polymers to the polyvalent ion, valence of one functional group is more preferably monovalent.

Examples of anionic functional groups of the ionic polymers may include carboxylic acid, sulfurous acid, sulfuric acid, acrylic acid, and salts thereof. Examples of cationic functional groups of the ionic polymers may include amine salt, imine salt and the like.

Examples of anionic ionic polymers may include polyacrylate, hexamethaphosphate, polycarboxylate (a polymer of carboxylate monomer) and the like. Examples of canionic ionic polymers may include polymers having a quaternary ammonium group and the like.

The molecular weight of the ionic polymer is preferably 3,000 or more, more preferably 5,000 or more, and further more preferably 6,000 or more. If the molecular weight is less than 3,000, weak flocculation cannot be formed. The molecular weight of the ionic polymer is preferably 15,000 or less, more preferably 12,000 or less, and futher more preferably 10,000 or less. If the molecular weight exceeds 15,000, stickiness as a feeling of use will occur.

The content of the salt (polyelectrolyte) that is a source of the ionic polymer in the composition is preferably 0.005 part by mass or more, more preferably 0.008 part by mass or more, more preferably 0.01 part by mass or more, more preferably 0.015 part by mass or more, and further more preferably 0.02 part by mass or more with resect to 1 part by mass of the powder. If the content of the polyelectrolyte is less than 0.005 part by mass, dispersibility of the powder will deteriorate. The content of the salt (polyelectrolyte) that is a source of the ionic polymer is preferably 0.5 part by mass or less, more preferably 0.4 part by mass or less, and further more preferably 0.3 part by mass or less with resect to 1 part by mass of the powder. If the content of the polyelectrolyte exceeds 0.5 part by mass, feeling of use will be adversely affected.

### [Polyvalent ion]

The composition of the present disclosure comprises at least one kind of a polyvalent ion. The polyvalent ion is an ion having the first charge in a solvent. The polyvalent ion is preferably an ion having valence of two or more.

The polyvalent ion may be a metal ion, for example. Examples of the polyvalent ion as a cation may include metal ions of aluminum, magnesium, calcium, manganese, barium, nickel, iron, zinc, copper, bismuth, tin, silver and the like. Examples of the polyvalent ion as an anion may include sulfate ion, carbonate ion and the like. Among them, magnesium ion and calcium ion are preferred in particular from the viewpoint of safety.

In cases where the polyvalent ion is a metal ion, the polyvalent ion can be added in a form as a metal salt. The metal salt may be one that ionizes (dissolves) in the solvent. Examples of the metal salt may include metal salts such as chloride salt, hydroxide salt, nitrate, sulfate, phosphate, acetate, carbonate and the like. These metal salts can be used alone or by combining two or more kinds thereof.

The content of the salt that is a source of the polyvalent ion in the composition of the present disclosure is preferably 0.05 part by mass or more, more preferably 0.08 part by mass or more, more preferably 0.1 part by mass or more, more preferably 0.15 part by mass or more, and further more preferably 0.2 part by mass or more with respect to 1 part by mass of the polyelectrolyte that is a source of the ionic polymer. If the content of the salt that is a source of the polyvalent ion is less than 0.05 part by mass, effect for enhancing redispersibility cannot be achieved. The content of the salt that is a source of the polyvalent ion is preferably 5 parts by mass or less, more preferably 3 parts by mass or less, and further more preferably 2 parts by mass or less with respect to 1 part by mass of the polyelectrolyte that is a source of the ionic polymer. If the content of the polyvalent ion exceeds 5 parts by mass, stickiness will occur. The viscosity of the composition may be increased when the content of the polyvalent ion is increased.

The composition of the present disclosure may comprise an electrifying agent (charging agent) that charges the surface of the powder with the first charge when the powder is not charged in the solvent. Cationic polymers such as polyethylene imine and the like, for example, may be used as the electrifying agent to positively charge the surface of the powder. Anionic polymers such as polyacrylate and the like, for example, may be used as the electrifying agent to negatively charge the surface of the powder.

### [Other components]

The composition of the present disclosure may include, as appropriate and as necessary, other components within the range of not inhibiting the effect of the present disclosure such as esters, anionic surfactants, cationic surfactants, amphoteric surfactants, nonionic surfactants, moisturizers, water-soluble polymers, thickeners, film-forming agents, ultraviolet absorbers, sequestrants, amino acids, organic amines, polymeric emulsions, pH adjusters, skin nutrients, vitamins, antioxidants, antioxidant aids, perfumes, water and the like.

Examples of other components that can be blended are listed in the following. At least one of the components listed below can be added to the composition of the present disclosure.

Examples of the anionic surfactants that may be used may include fatty acid soap (such as sodium laurate and sodium palmitate); higher alkyl sulfate ester salt (such as sodium lauryl sulfate and potassium lauryl sulfate); alkyl ether sulfate ester salt (such as POE-lauryl sulfate triethanolamine and sodium POE-lauryl sulfate); N-acyl sarcosinic acid (such as sodium lauroyl sarcosinate); higher fatty acid amide sulfonate (such as sodium N-myristoyl-N-methyltaurate, sodium methyl cocoyl taurate and sodium laurylmethyl taurate); phosphate ester salt (sodium POE-oleylether phosphate and POE-stearylether phosphate); sulfosuccinate (such as sodium di-2-ethylhexyl sulfosuccinate, sodium monolauroyl monoethanolamide polyethylene sulfosuccinate and sodium lauryl polypropylene glycol sulfosuccinate); alkylbenzene sulfonate (such as sodium linear dodecylbenzene sulfonate, triethanolamine linear dodeylbenzene sulfonate, and linear dodecylbenzene sulfonate); higher fatty acid ester sulfate ester salt (such as sodium hydrogenated gryceryl cocoate sulfate); N-acyl glutamate (such as monosodium N-lauroyl glutamate, disodium N-stearoyl glutamate and monosodium N-myristoyl-L-glutamate); sulfonated oil (such as Turkey red oil); POE-alkyl ether carboxylic acid; POE-alkyl aryl ether carboxylate; α-olefin sulfonate; higher fatty acid ester sulfonate; secondary alcohol sulfate ester salt; higher fatty acid alkylolamide sulfate ester salt; sodium lauroyl monoethanolamide succinate; N-palmitoyl asparaginate ditriethanolamine; sodium casein; and the like.

Examples of the cationic surfactants may include alkyltrimethyl ammonium salt (such as stearyltrimethyl ammonium chloride, lauryltrimethyl ammonium chloride); alkylpyridinium salt (such as cetylpyridinium chloride); distearyldimethyl ammonium chloride; dialkyldimethyl ammonium salt; poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE alkylamine; alkylamine salt; polyamine fatty acid derivative; amyl alcohol fatty acid derivative; benzalkonium chloride; benzethonium chloride and the like.

Examples of the amphoteric surfactant that may be used may include: imidazoline-based amphoteric surfactant (such as sodium 2-undecyl-N,N,N-(hydroxyethylcarboxymethyl)-2-imidazoline and 2-cocoyl-2-imidazolinium hydroxide-1-carboxyethyloxy disodium salt); and betaine-based surfactant (such as 2-heptadecyl-N-carboxymethyl-N-hydroxyethyl imidazolinium betaine, lauryl dimethylaminoacetic acid betaine, alkyl betaine, amidobetaine, and sulfobetaine).

Examples of the lipophilic nonionic surfactants may include sorbitan fatty acid ester (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate, etc); glyceryl polyglyceryl fatty acid (such as glyceryl monocotton oil fatty acid, glyceryl monoerucate, glyceryl sesquioleate, glyceryl monostearate, glyceryl α, α'-oleate pyroglutamate, glyceryl monostearate malate, etc); propylene glycol fatty acid ester (such as propylene glycol monostearate, etc); hydrogenated caster oil derivative; glyceryl alkyl ether and the like.

Examples of the hydrophilic nonionic surfactants that may be used may include POE sorbitan fatty acid ester (such as POE sorbitan monooleate, POE sorbitan monostearate, POE sorbitan monooleate, POE sorbitan tetraoleate); POE sorbit fatty acid ester (such as POE sorbit monolaurate, POE sorbit monooleate, POE sorbit pentaoleate, POE sorbit monostearate), POE glyceryl fatty acid ester (such as POE monooleate such as POE glyceryl monostearate, POE glyceryl monoisostearate, POE glyceryl triisostearate); POE fatty acid ester (such as POE distearate, POE monodioleate, ethyleneglycol distearate); POE alkyl ether (such as POE lauryl ether, POE oleyl ether, POE stearyl ether, POE behenyl ether, POE-2-octyldodecyl ether, POE cholestanol ether); puluronic type (such as Puluronic), POE/POP alkyl ethers (such as POE/POP cetyl ether, POE/POP 2-decyltetradecyl ether, POE/POP monobutyl ether, POE/POP hydrogenated lanoline, POE/POP glycerin ether); tetra POE/tetra POP ethylenediamine condensation products (such as Tetronic); POE castor oil hydrogenated castor oil derivative (such as POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivative (such as POE sorbitol beeswax); alkanolamide (such as coconut oil fatty acid diethanolamide, lauric acid monoethanolamide, fatty acid isopropanolamide); POE propyleneglycol fatty acid ester; POE alkyl amines; POE fatty acid amide; sucrose fatty acid ester; alkylethoxydimethylamine oxide; trioleyl phosphoric acid and the like.

Examples of the moisturizers may include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salt, dl-pyrrolidone carboxylate, alkyleneoxide derivative, short-chain soluble collagen, diglycerin (EO)PO adduct, chestnut rose extract, yarrow extract, melilot extract and the like.

Examples of the natural water-soluble polymer may include plant-based polymer (such as gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat),glicyrrhizic acid); microorganism based polymer (such as xanthan gum, dextran, succinoglycan, pullulan, etc), animal-based polymer (such as collagen, casein, albumin, gelatine, etc) and the like.

Examples of the semisynthetic water-soluble polymer may include starch-based polymer (such as carboxymethyl starch, methylhydroxypropyl starch, etc); cellulose-based polymer (such as methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, crystalline cellulose, cellulose powder, etc); algin acid-based polymer (such as sodium alginate, propylene glycol alginate ester, etc.) and the like.

Examples of the synthetic water-soluble polymer may include vinyl based polymer (such as polyvinyl alcohol, polyvinyl methyl ether, polyvinylpyrrolidone, carboxyvinylpolymer, etc); polyoxyethylene based polymer (such as polyoxyethylenepolyoxypropylene copolymer such as polyethylene glycol 20,000, 40,000 and 60,000, etc); acrylic polymer (such as sodium polyacrylate, polyethylacrylate, polyacrylamide, etc); polyethyleneimine; cationic polymer and the like.

Examples of thickeners may include gum arabic, carrageenan, karaya gum, tragacanth gum, carob gum, quince seed (marmelo), casein, dextrin, gelatin, sodium pectate, sodium alginate, methylcellulose, ethyl cellulose, CMC, hydroxyethyl cellulose, hydroxypropyl cellulose, PVA, PVM, PVP, sodium polyacrylate, carboxyvinyl polymer, locustbean gum, guar gum, tamarind gum, dialkyldimethylammonium sulfate cellulose, xanthan gum, magnesium aluminum silicate, bentonite, hectorite, magnesium aluminum silicate (veegum), laponite, silicic anhydride and the like.

Examples of the film-forming agents may include an anionic film-forming agent (such as (meta)acrylic acid/(meta)acrylic acid ester copolymer, methyl vinyl ether/maleic anhydride coplymer, etc), a cationic film-forming agent (such as cationic cellulose, diallyldimethylammonium chloride polymer, diallyldimethylammonium chloride/acrylic amide copolymer, etc), a nonionc film-forming agent (such as polyvinyl alcohol, polyvinylpyrrolidone, polyvinyl acetate, polyacrylic ester copolymer, (meta)acrylamide, polymeric silicone, silicone resin, trimethylsiloxysilicate, etc.) and the like.

Examples of the ultraviolet light absorbers may include benzoic acid family ultraviolet light absorber (such as p-aminobenzoic acid (hereinafter abbreviated as PABA), PABA monoglycerine ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc); anthranilic acid family ultraviolet light absorber (such as homomenthyl N-acetylanthranilate etc); salicylic acid family ultraviolet light absorber (such as amyl salicylate, menthyl salicylate, homomenthyl salicylate, octyl salicylate, phenyl salicylate, benzyl salicylate, p-isopropanolphenyl salicylate, etc); cinnamic acid family ultraviolet light absorber (such as octyl methoxycinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate, glyceryl mono-2-ethylhexanoyl-diparamethoxy cinnamate, etc); benzophenone family ultraviolet light absorber (such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc); 3-(4'-methylbenzylidene)-d,1-camphor and 3-benzylidene-d,1-camphor; 2-phenyl-5-methylbenzoxazol; 2,2'-hydroxy-5-methylphenylbenzotriazol, 2-(2'-hydroxy-5'-t-octylphenyl) benzotriazol, 2-(2'-hydroxy-5'-methylphenylbenzotriazol; dibenzalazine; dianisoylmethane; 4-methoxy-4'-t-butyldibenzoylmethane; 5-(3,3-dimethyl-2-norbornylidene)-3-pentane-2-one; dimorpholinopyridazinone; 2-ethylhexyl 2-cyano-3,3-diphenylacrylate; 2,4-bis-{[4-(2-ethylhexyloxy)-2-hydroxy]-phenyl}-6-(4-methoxyphenyl)-(1,3,5)-tria zine and the like.

Examples of the sequestrants may include 1-hydroxyethane-1, 1-diphosphonic acid, 1-hydroxyethane, 1-diphosphonic acid 4Na salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate and the like.

Examples of the amino acids may include neutral amino acid (such as threonine, cysteine, etc); basic amino acid (such as hydroxylysine, etc) and the like. Examples of the amino acid derivative may include sodium acyl sarcosinate (sodium lauroyl sarcosinate), acyl glutamate, sodium acyl β-alanine, glutathione, pyrrolidone carboxylate and the like.

Examples of the organic amines may include monoethanolamine, diethanolamine, triethanolamine, morpholine, triisopropanolamine, 2-amino-2-methyl-1,3-propanediol, 2-amino-2-methyl-1-propanol and the like.

Examples of the polymer emulsions may include acrylic resin emulsion, ethyl polyacrylate emulsion, solution of acrylic resin, polyacrylalkylester emulsion, polyvinyl acetate resin emulsion, natural rubber latex and the like.

Examples of the pH adjusters may include buffer such as lactic acid-sodium lactate, citric acid-sodium citrate, succinic acid-sodium succinate and the like.

Examples of the vitamins may include vitamine A, B1, B2, B6, C, E and derivatives thereof, pantothenic acid and derivatives thereof, biotin and the like.

Examples of the anti-oxidants may include tocopherols, dibutyl hydroxy toluene, butyl hydroxy anisole, and gallic acid esters and the like.

Examples of the anti-oxidant aids may include phosphoric acid, citric acid, ascorbic acid, maleic acid, malonic acid, succinic acid, fumaric acid, cephalin, hexamethaphosphate, phytic acid, ethylenediaminetetraacetic acid and the like.

Examples of other containable compositions may include an antiseptic agent (such as ethylparaben, butylparaben, chlorphenesin, 2-phenoxyethanol, etc.); antiphlogistic (such as glycyrrhizinic acid derivatives, glycyrrhetic acid derivatives, salicylic acid derivatives, hinokitiol, zinc oxide, allantoin, etc.); a skin-whitening agent (such as placental extract, saxifrage extract, arbutin, etc.); various extracts (such as chestnut rose extract, phellodendron bark (cork tree bark), coptis rhizome, lithospermum, glycyrrhiza glabra, peony, swertia herb, birch, sage, loquat, carrot, aloe, okra, mallow, iris, grape, coix seed, rubus suavissimus leaf, sponge gourd, lily, prunus yedoensis leaf, saffron, cnidium rhizome, ginger, hypericum, restharrow, garlic, red pepper, citrus unshiu, Japanese angelica, green tea, seaweed, yeast, etc.); an activator (such as royal jelly, photosenstizer, cholesterol derivatives, etc.); a blood circulation accelerator (such as nonylic acid vanillylamide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc); an antiseborrheric agent, (such as sulfur, thianthl, etc); an anti-inflammatory agent (such as tranexamic acid, thiotaurine, hypotaurine, etc.) and the like.

Furthermore, the composition of the present disclosure may include, as necessary, caffeine, tannin, verapamil, tranexamic acid and derivatives thereof; various crude drug extracts such as licorice, Chinese quince, Pyrola japonica and the like; drugs such as tocopherol acetate, glycyrrhetinic acid, glycyrrhizic acid and derivatives thereof, or salts thereof; skin-whitening agents such as vitamin C, magnesium ascorbyl phosphate, ascorbic acid glucoside, arbutin, kojic acid and the like; amino acids such as arginine and lysine and the like and derivatives thereof.

### [Dispersing mechanism]

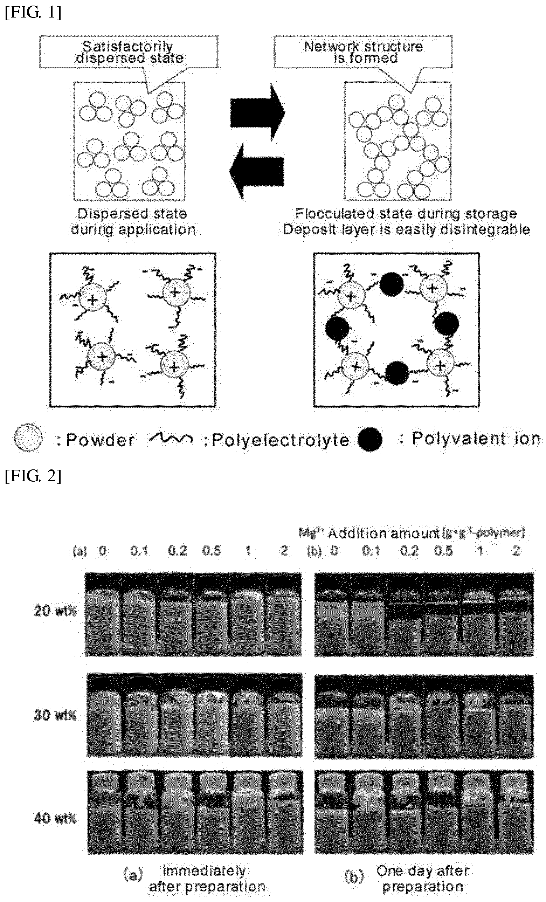

Figure 1 is a schematic view showing a dispersed state in the composition of the present disclosure. In the solvent, the surface of the powder has a first charge (a positive charge in Figure 1). The ionic polymer is ionized in the solvent and has a second charge (a negative charge in Figure 1). It is considered that polymeric ions gather near the surface of the powder to maintain electrical neutrality. In the solvent, it is considered that polymeric ions are electrostatically and/or ionically bonded to the surface of the powder. Alternatively, it is considered that ionic polymers are attached or adsorbed to the surface of the powder. Accordingly, it is considered that the powder and the ionic polymer form a complex. It is considered that a plurality of ionic polymers are bonded or attached to one primary particle and/or secondary particle of the powder. It is considered that, in the complex, a polymer chain of ionic polymers is not contracted around the powder and is stretched outward from the powder. When the composition of the present disclosure is to be used (upon application, for example) or the powder is dispersed by shaking the container, it is considerd that the powder is dispersed as the complex in a state shown in the figure on the left side of Figure 1.

The average particle size of the complex may be 200 nm or greater. Moreover, the average particle size of the complex may be 800 nm or smaller. The average particle size of the complex can be measured in accordance with a dynamic light scattering method. The polydispersity index (PDI) of the complex is preferably 0.2 or less. The polydispersity index can be calculated by particle size distribution measured in accordance with a dynamic light scattering method.

When the composition of the present disclosure is left to stand, it is considered that the polyvalent ion is electrostatically and/or ionically bonded to the ionic polymer having the second charge in the solvent as shown in the figure on the right side of Figure 1. Moreover, it is considered that the polyvalent ion is electrostatically and/or ionically bonded to a plurality of the ionic polymers adsorbed to different powders. Accordingly, it is considered that the polyvalent ion has a structure so as to crosslink the ionic polymer or the complex. That is, it is considered that the powder, the ionic polymer and the polyvalent ion form a network by weak ionic bond or ionic and/or electrostatic interaction. It is considered that the powder weakly flocculates via the ionic polymer and the polyvalent ion. Furthermore, it is considered that the network and/or weak flocculation suppresses sedimentation of the powder and suppresses solidification even if the powder sediments. Moreover, it is considered that the powder after being left to stand can be easily reflowed and redispersed by external force such as shaking since the network and/or weak flocculation can be easily decomposed by external force.

As stated above, according to the composition of the present disclosure, sedimentation of the powder can be suppressed. Moreover, solidification of the powder can be suppressed even if the powder is left to stand and sediments. Furthermore, the powder can be easily reflowed and redispersed by stirring or shaking the container.

A cosmetic of the present disclosure according to a second embodimenr will be described.

The cosmetic of the present disclosure comprises the composition according to the first embodiment. The cosmetic may comprise an additive as desired.

The cosmetic of the present disclosure has an effect similar to the composition according to the first embodiment.

A method of producing the composition and the cosmetic of the present disclosure will be described as a third embodiment. The method described below is merely an example, and the production method is not limited thereto. Reference should be made to the aforementioned description for details of each component.

First of all, the powder of which the surface is to be charged with the first charge in the solvent is added to the solvent (a first addition step). The surface of the powder is charged if necessary (a charging step). For example, pH may be adjusted. Alternatively, the surface of the powder may be charged by adding a charging agent to the solvent. For example, cationic polymers such as polyethylene imine and the like can be added when the surface of the powder needs to be charged positively.

Next, a polyelectrolyte that generates the ionic polymer having the second charge is added to the solvent (a second addition step). Accordingly, it is considered that the ionic polymer generated by ionization of the polyelectrolyte electrically adsorbs to the surface of the powder.

With respect to the order of addition of the powder and the polyelectrolyte, it is preferred that addition of the powder is first. The ionic polymer can be adsorbed to the powder easily by adding the ionic polymer in a state where the powder is dispersed first.

A process for loosening contraction of the polymer chain of the ioinic polymer may be performed (a loosening step). For example, stress such as pressure and strain may be applied to the liquid so that entanglement of the polymer chain may be disentangled and the polymer chain may stretch outward from the particle which adsorbs the polymer chain. The application time of pressure to the liquid is preferably one hour or longer so that loosening of the polymer chain may be advanced sufficiently. For example, contraction of the polymer chain can be loosened by sealing the container of which the liquid is contained after applying pressure to the liquid.

Next, a compound (a salt, for example) that generates the polyvalent ion having the first charge by ionization, for example, is added to the aforementioned mixture (a third addition step). It is considered that the polyvalent ion generated in the solvent ionically and/or electrostatically bonds or weakly bonds with the ionic polymer. Accordingly, it is considered that the powder weakly flocculates via the ionic polymer and the polyvalent ion.

The addition rate of each component can be determined in accordance with the aforementioned contents.

Other components can be added in each step so that the effect of the present disclosure is not inhibited.

According to the third embodiment, the compositon and the cosmetic of the present disclosure can be prepared easily. In cases where the powder, the ionic polymer and the polyvalent ion are added simultaneously, it is considered that polymer chain of the ionic polymer adsorbed to the powder contracts. Accordingly, the polyvalent ion cannot connect the ionic polymers; as a result, a network is not formed and the powder cannot form weak flocculation. On the other hand, according to the method of the present disclosure, it is considered that the polymer chain of the ionic polymer adsorbed to the powder can be streched. Accordingly, the polyvalent ion can connect the ionic polymers; as a result, a network is formed and the powder can form weak flocculation.

### Examples

Examples of the composition of the present disclosure will be described below. The composition of the present disclosure, however, is not limited to the following examples. The contents of the ionic polymer shown in each table are the addition rates of the polyelectrolyte that is the basis of the ionic polymer. The contents of the polyvalent ion shown in each table are the addition rates of the salt that is the basis of the polyvalent ion.

### [Test Example 1: Test Examples 1-1 to 1-6], [Test Example 2: Test Examples 2-1 to 2-6] and [Test Example 3: Test Examples 3-1 to 3-6]

Compositions of which the concentrations of the powder and the polyvalent ion were varied were prepared, and dispersibility and redispersibility thereof were evaluated. Tables 1-3 show formulations of the prepared compositions and evaluations for dispersibility and redispersibility.

Water was used as the solvent. Zinc oxide powder having the average particle size of 0.05 µm (catalogue value) was added to water as the powder. It is considered that the surface of zinc oxide particle is positively charged in water. Then, ammonium polyacrylate that is a polyelectrolyte was added to water to disperse zinc oxide powder by applying ultrasonic vibration to the mixture. Ammonium polyacrylate ionizes in water, and the ionic polymer generated is a polyacrylate ion having a carboxylate anionic group having a negative charge. The addition rates of the "polyelectrolyte (ionic polymer)" shown in Tables 1-3 are the masses of the polyelectrolyte per 1 g of the powder. Then, magnesium chloride as an electrolyte (a salt) that becomes the polyvalent ion was added to the mixture and mixed. The addition rates of the "salt (polyvalent ion)" shown in Tables 1-3 are the masses of the electrolyte (salt) per 1 g of the polyelectrolyte.

With respect to each composition, dispersibility of the powder was confirmed immediately after preparation and one day after preparation. Figure 2 shows photographs of the compositions immediately after preparation and one day after preparation of each test example.

Dispersibility was visually evaluated on the following criteria.
A: The powder is dispersed uniformly.
B: The powder sediments, but the boundary between the solvent and the deposition layer is vague and suspension of the powder is observed at the upper part of the deposition layer.
C: The powder sediments, the boundary between the solvent and the deposition layer is clear and the solvent above the deposition layer is clear.

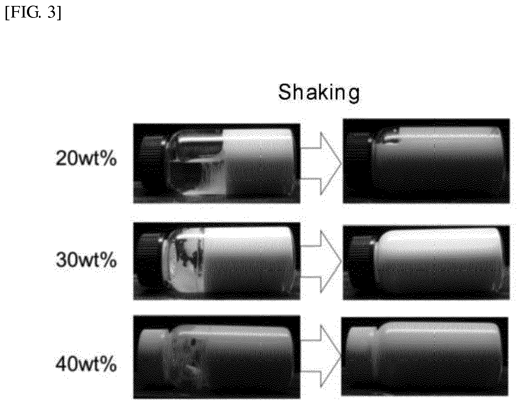

The compositions after one day from preparation were confirmed for redispersibility of the powder. The deposition layer of zinc oxide was shaken or agitated to visually evaluate whether zinc oxide can flow based on the following criteria. Figure 3 shows photographs of which the container was shaken by hand with respect to the samples after one day from preparateion in Test Examples 1-3, 2-3 and 3-3.
A: The deposition layer of zinc oxide was flowable easily.
B: The deposition layer of zinc oxide was flowabl, but dispersibility was poor.
C: The deposition layer of zinc oxide was hardly flowable.

In any content of the powder, the particles that were dispersed immediately after preparation sedimented after one day in the condition where the addition rate of Mg²⁺ was 0.2 g/(g-polymer) or more. In particular, a clear layer was not formed even if the slurry sedimented in the condition where the powder was 30% by mass and 40% by mass.

In Test Examples 1-1, 2-1 and 3-1 of which magnesium ion was not added, the viscosity of the deposition layer of zinc oxide was high and good dispersibility could not be achieved. In Test Examples 1-2, 2-2 and 3-2 of which 0.1 g of magnesium chloride that is a source of of a magnesium ion was added with respect to 1 g of ammonium polyacyrylate that is a source of a polyacrylate ion, zic oxide was flowable but redispersibility was poor. On the other hand, in Test Examples 1-3 to 1-6, 2-3 to 2-6 and 3-3 to 3-6 of which 0.2 g or more of magnesium chloride was added with respect to 1 g of ammonium polyacrylate, the deposition layer of zinc oxide could be easily flown by shaking by hand and good redispersibility could be achieved, as shown in Figure 3.

Accordingly, it has been found that dispersibility and redispersibility of the powder can be enhanced by adding the ionic polymer and the polyvalent ion even if the content of the powder is as high as 10% by mass to 50% by mass, and at least 20% by mass to 40% by mass.

It has been found that reflowability and redispersibility of the powder can be enhanced if the content of the salt that is a source of the polyvalent ion is at least 0.05 part by mass or more, preferably 0.1 part by mass or more, and more preferably 0.15 part by mass or more with respect to 1 part by mass of the polyelectrolyte.

**[Table 1]**

| | Powder (mass %) | Polyelectrolyte (Ionic Polymer) (mg/g(particle)) | Salt (Polyvalent Ion) (g/g(polymer)) | Immediately After Preparation | One Day After Preparation | |
|---|---|---|---|---|---|---|
| | | | | Dispersibility | Dispersibility | Redispersibility |
| Test Example 1-1 | 20 | 10 | 0 | A | B | C |
| Test Example 1-2 | | | 0.1 | A | B | B |
| Test Example 1-3 | | | 0.2 | A | C | A |
| Test Example 1-4 | | | 0.5 | A | C | A |
| Test Example 1-5 | | | 1 | A | C | A |
| Test Example 1-6 | | | 2 | A | C | A |

**[Table 2]**

| | Powder (mass %) | Polyelectrolyte (Ionic Polymer) (mg/g(particle)) | Salt (Polyvalent Ion) (g/g(polymer)) | Immediately After Preparation | One Day After Preparation | |
|---|---|---|---|---|---|---|
| | | | | Dispersibility | Dispersibility | Redispersibility |
| Test Example 2-1 | 30 | 10 | 0 | A | B | C |
| Test Example 2-2 | | | 0.1 | A | B | B |
| Test Example 2-3 | | | 0.2 | A | C | A |
| Test Example 2-4 | | | 0.5 | A | C | A |
| Test Example 2-5 | | | 1 | A | C | A |
| Test Example 2-6 | | | 2 | A | C | A |

**[Table 3]**

| | Powder (mass%) | Polyelectrolyte (Ionic Polymer) (mg/g(particle)) | Salt (Polyvalent Ion) (g/g(polymer)) | Immediately After Preparation | One Day After Preparation | |
|---|---|---|---|---|---|---|
| | | | | Dispersibility | Dispersibility | Redispersibility |
| Test Example 3-1 | 40 | 10 | 0 | B | C | C |
| Test Example 3-2 | | | 0.1 | B | C | B |
| Test Example 3-3 | | | 0.2 | B | C | A |
| Test Example 3-4 | | | 0.5 | B | C | A |
| Test Example 3-5 | | | 1 | B | C | A |
| Test Example 3-6 | | | 2 | B | C | A |

### [Test Example 4]

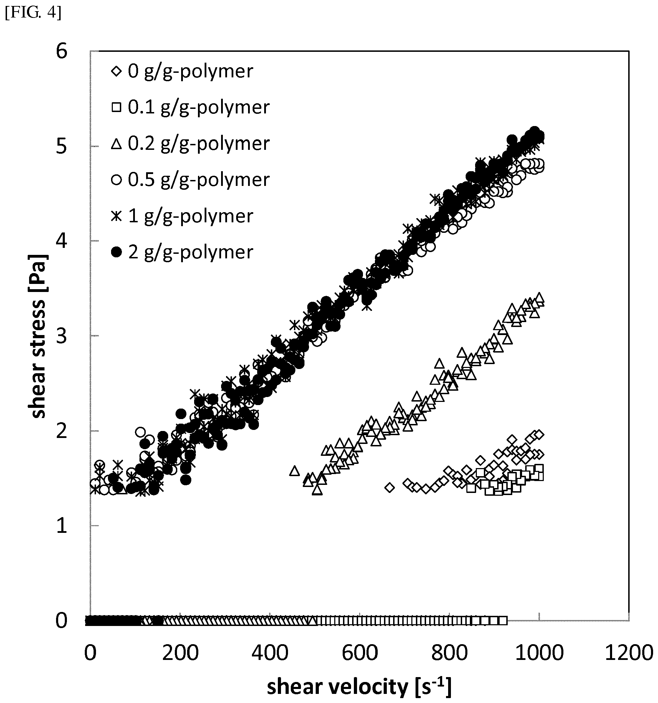

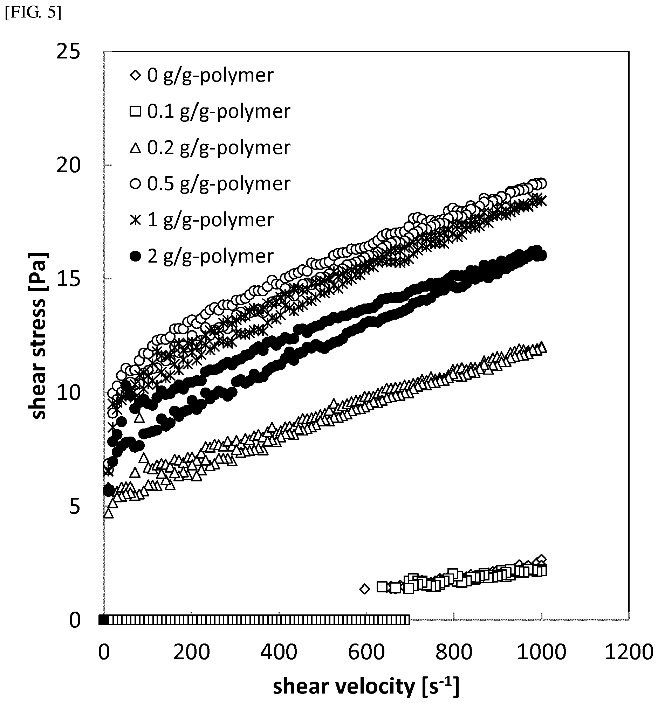

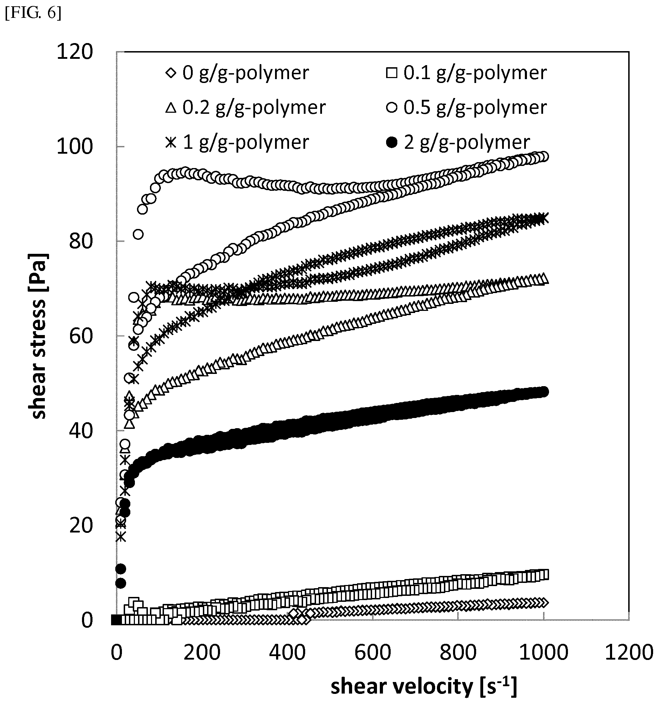

In order to evaluate flowability of the composition of the present disclosure quantitatively, rheological properties of the compositions of each Test Examples in Test Examples 1-1 to 1-6, 2-1 to 2-6 and 3-1 to 3-6 were measured by a cone/plate rheometer. Figures 4 to 6 show relationship between the shear velocity and the shear stress. Figure 4 is a graph related to Test Examples 1-1 to 1-6. Figure 5 is a graph related to Test Examples 2-1 to 2-6. Figure 6 is a graph related to Test Examples 3-1 to 3-6.

Looking at the lines of continuing points in Figures 4 to 6, each line crosses the vertical axis and a certain shear stress is present when the shear velocity is 0/s. That is, it can be seen that each composition has a yield value. The yield value indicates a force required for the powder in the composition to start moving. In Figure 4, for example, it means that the powder in the composition having the content of the salt (polyvalent ion) of 0.2 to 2 g/g-polymer needs about 1.5 Pa to start moving. Accordingly, it is shown that the deposition layer of zinc oxide behaves like solid in a standing state. On the other hand, it is shown that inclination, i.e. viscosity, is low and the deposition layer of zinc oxide can be easily flown in a state where shear is applied. Accordingly, it is shown that the powders in the compositions of Test Examples 1-3 to 1-6, 2-3 to 2-6 and 3-3 to 3-6 behave as solid in a standing state and behave as fluid upon use. It is considered that the powder does not form weak flocculation in the composition having the content of the salt (polyvalent ion) of 0 to 0.1 g/g-polymer.

Furthermore, in Figures 4-6, it can be seen that the inclinations of the lines of continuing points are changed when the addition amounts of the polyvalent ion are changed. Accordingly, the viscosity upon flowing can be adjusted by adjusting the addition amount of the polyvalent ion.

Furthermore, when hysteresis measurement that reduces the shear velocity after increasing the shear velocity is performed, the yield values at the beginning and the end of the measurement were almost the same value in any measurement. This indicates that a similar network configuration can be formed again even if the composition is left to stand after agitating. In other words, a slurry that has high stability upon storing and flows upon use can be obtained.

The composition and cosmetic of the present invention, and the producing method thereof have been described according to the foregoing embodiments and examples, but the invention is not limited to the foregoing embodiments and examples and may encompass various transformations, modifications, and improvements made to the various disclosed elements (including elements disclosed in the Claims, Description, and Drawings) within the scope of the invention and according to the fundamental technical idea of the present invention. Further, various combinations, substitutions, and selections of the various disclosed elements are possible within the scope of the claims of the invention.

Further issues, objectives, and embodiments (including modifications) of the present invention are revealed also from the entire disclosure of the invention including the Claims.

The numerical ranges disclosed herein are to be construed in such a manner that arbitrary numerical values and ranges falling within the disclosed ranges are treated as being concretely described herein, even where not specifically stated.

### Industrical Applicability

The composition of the present disclosure can be generally applied to products that uses slurry. For example, the composition of the present disclosure can be applied to cosmetics, paints, inks, foods and the like.

## Claims

1. A composition comprising:
a solvent;
a powder that is present in the solvent and bears a first charge on a surface;
an ionic polymer having a second charge that is opposite to the first charge; and
a polyvalent ion having the first charge;
wherein the powder and the ionic polymer are electrostatically and/or ionically bonded with each other; and
the ionic polymer and the polyvalent ion are electrostatically and/or ionically bonded with each other.

2. The composition according to claim 1, wherein the powder forms a weak flocculation via the ionic polymer and the polyvalent ion.

3. The composition according to claim 1 or 2, wherein an average particle size of a complex having the powder and the ionic polymer attached to the powder is 200 nm to 800 nm.

4. The composition according to any one of claims 1 to 3, wherein the first charge is a positive charge, and the second charge is a negative charge.

5. The composition according to any one of claims 1 to 4, wherein the powder is zinc oxide.

6. The composition according to any one of claims 1 to 5, wherein a content of the powder is 10% by mass to 50% by mass with respect to a mass of the composition.

7. The composition according to any one of claims 1 to 6, wherein the average particle size of the powder is 10 nm to 200 nm.

8. The composition according to any one of claims 1 to 7, wherein the ionic polymer has an anionic functional group.

9. The composition according to any one of claims 1 to 8, wherein the ionic polymer is a polyacrylate ion and/or a hexametaphosphate ion.

10. The composition according to any one of claims 1 to 9, wherein a content of a polyelectrolyte that is a source of the ionic polymer is 0.005 part by mass to 0.5 part by mass with respect to 1 part by mass of the powder.

11. The composition according to any one of claims 1 to 10, wherein the polyvalent ion is a magnesium ion and/or a calcium ion.

12. The composition according to any one of claims 1 to 11, wherein a content of a salt that is a source of the polyvalent ion is 0.05 part by mass to 5 parts by mass with respect to 1 part by mass of the polyelectrolyte that is a source of the ionic polymer.

13. The composition according to any one of claims 1 to 12, wherein the solvent comprises water.

14. A composition comprising:
a powder bearing a first charge on a surface;
a polyacrylate ion and/or a hexametaphosphate ion; and
a magnesium ion;
wherein a content of the powder is 10% by mass to 50% by mass with respect to the composition,
a content of a polyelectrolyte that is a source of the polyacrylate ion and/or the hexametaphosphate ion is 0.005 part by mass to 0.5 part by mass with respect to 1 part by mass of the powder, and
a content of a salt that is a source of the magnesium ion is 0.05 part by mass to 5 parts by mass with respect to 1 part by mass of the polyelectrolyte.

15. The composition according to claim 14, wherein the powder is zinc oxide.

16. A cosmetic comprising the composition according to claims 1 to 15.

17. A method of producing a composition, comprising:
a first addition step of adding a powder to a solvent comprising water, the powder bearing a first charge on a surface in the solvent;
a second addition step of adding a polyelectrolyte to a solvent, wherein the polyelectrolyte ionizes an ionic polymer having a second charge opposite to the first charge in the solvent; and
a third addition step of adding a salt to the solvent after the first and the second addition steps, wherein the salt generates a polyvalent ion having the first charge in the solvent.

18. A method of producing a composition, comprising:
a first addition step of adding a powder to a solvent comprising water, the powder bearing a first charge on a surface in the solvent;
a second addition step of adding a polyelectrolyte to the solvent, wherein the polyelectrolyte ionizes an ionic polymer having a second charge opposite to the first charge in the solvent;
a charging step of charging the surface of the powder with the first charge in the solvent; and
a third addition step of adding a salt to the solvent after the charging step, wherein the salt generates a polyvalent ion having the first charge in the solvent.

19. The method according to claim 18, wherein pH of the solvent is adjusted in the charging step.

20. The method according to claim 18 or 19, wherein a charging agent that charges the surface of the powder with the first charge is added to the solvent in the charging step.

21. The method according to claim 20, wherein the charging agent is a cationic polymer.

22. The method according to any one of claims 17 to 21, further comprising: a loosening step of loosening contraction of a polymer chain of the ionic polymer.

23. The method according to claim 22, wherein pressure is applied to the solvent in the loosening step.

24. The method according to any one of claims 17 to 23, wherein the powder is zinc oxide.

25. The method according to any one of claims 17 to 24, wherein an average particle size of the powder is 10 nm to 200 nm.

26. The method according to any one of claims 17 to 25, wherein the polyelectrolyte is polyacrylate and/or hexametaphosphate.

27. The method according to any one of claims 17 to 26, wherein the salt is a magnesium salt and/or a calcium salt.
